# EUROPÄISCHE PATENTSCHRIFT

(11) **EP 1 299 457 B1**
(45) Veröffentlichungstag und Bekanntmachung des Hinweises auf die Patenterteilung: **17.10.2012**
(21) Anmeldenummer: 01945245.7
(22) Anmeldetag: 02.06.2001
(51) Int. Cl.: C08H 1/00, C07K 14/805, A61K 38/42

(54) **MIT BLUTPLASMA VERTRÄGLICHE, VERNETZTE UND MIT POLYALKYLENOXIDEN KONJUGIERTE SÄUGETIERHÄMOGLOBINE ALS KÜNSTLICHE MEDIZINISCHE SAUERSTOFFTRÄGER, IHRE HERSTELLUNG UND IHRE VERWENDUNG**
MAMMALIAN HAEMOGLOBIN COMPATIBLE WITH BLOOD PLASMA, CROSS-LINKED, AND CONJUGATED WITH POLYALKYLENE OXIDES AS ARTIFICIAL MEDICAL OXYGEN CARRIERS, PRODUCTION AND USE THEREOF
HEMOGLOBINES MAMMALIENNES COMPATIBLES AVEC DU PLASMA SANGUIN, RETICULEES, ET CONJUGUEES AVEC DES OXYDES DE POLYALKYLENE, SERVANT DE PORTEURS D'OXYGENE MEDICAUX ARTIFICIELS, ET FABRICATION ET UTILISATION DE CELLES-CI

(30) Priorität: 29.06.2000 DE 10031744
(43) Veröffentlichungstag der Anmeldung: 09.04.2003
(73) Patentinhaber: SanguiBioTech GmbH, 58455 Witten an der Ruhr (DE)
(72) Erfinder: BARNIKOL, Wolfgang, 55128 Mainz (DE)
(74) Vertreter: Müller, Claudia
(86) Internationale Anmeldenummer: PCT/EP2001/006322
(87) Internationale Veröffentlichungsnummer: WO 2002/000768

(56) Entgegenhaltungen:
- EP-A- 0 206 448
- WO-A1-02/00229
- WO-A1-02/00230
- WO-A1-91/07190
- TON T. HAI ET AL.: "Polymerization of Diaspirin Crosslinked Hemoglobin (DCLHb) with PEG Activated with Benzenesulfonate Bearing Electron-Withdrawing Groups" TETRAHEDRON, Bd. 55, 1999, Seiten 2147-2156, XP002183148
- T.T.HAI ET AL.: "Diaspirin Crosslinked Hemoglobin (DCLHb) Polymerization" ART. CELLS, BLOOD SUBS., AND IMMOB. BIOTECH., Bd. 22, Nr. 3, 1994, Seiten 923-931, XP001039974

## Beschreibung

Die Erfindung betrifft mit bifunktionellen Vemetzem, ausgewählt aus Butandiepoxid, Divinylsulfon, einem Diisocyanat, einem Di-N-Hydroxysuccinimidylester, einem Diimidoester oder einem Dialdehyd oder Glykolaldehyd, unter Einsatz eines bezogen auf monomeres Hämoglobin 3- bis 60-fachen molaren Überschusses kovalent vemetzte Säugetier-Hämoglobine, an die ein Polyalkylenoxid, dessen terminale Hydroxygruppe mit einem maskierenden Molekül maskiert ist und dessen Molekulargewicht (Molare Masse) zwischen 200 und 5.000 g/mol beträgt, kovalent angeknüpft ist. Solche Hämoglobine sind überreschenderweise mit Proteinen menschlichen und tierischen Plasmas bei sämtlichen im Gefäßsystem des Körpers möglichen Bedingungen verträglich. Die Erfindung betrifft femer die Herstellung dieser vemetzten und mit Polyalkylenoxiden verknüpften Hämoglobine, sowie ihre Verwendung als künstliche intravasale Sauerstoffträger im menschlichen oder tierischen Organismus, in einzelnen Organen, oder für biomedizinische Zwecke. Künstliche Sauerstoffträger sind Stoffe, die Sauerstoff in für einen Organismus geeigneter Weise reversibel binden und freisetzen, sowie die Sauerstofftransportfunktion des Blutes ersetzen oder unterstützen können. Pharmazeutische Zubereitungen von Lösungen oder Suspensionen künstlicher Sauerstoffträger werden entwickelt, um zur Behandlung akuter und chronischer Sauerstoffmangelzustände sowie akuter Blutveriuste Menschen oder Tieren parenteral ins Gefäßsystem, insbesondere intravenös, verabreicht zu werden. Darüber hinaus können sie auch zur Perfusion von Organtransplantaten oder als Zusatz von Zellkulturen eingesetzt werden, um hier die Sauerstoffversorgung zu verbessern.
Künstliche Sauerstoffträger aus Hämoglobinen werden weltweit auf der Grundlage unterschiedlicher Konzepte entwickelt (Stand der Technik: Rudolph A. S. et al. (Hrsg.): Red Blood Cell Substitutes: Basic Principles and Clinical Applications, Marcel Dekker, New York u. a. 1998; Tsuchida E. (Hrsg.): Blood Substitutes: Present and Future Perspectives, Elsevier Science, Amsterdam 1998; Chang T. M. S. (Autor bzw. Hrsg.): Blood Substitutes: Principles, Methods, Products and Clinical Trials, Volume 1 und - Volume 2, Karger Landes, Basel u. a. 1997 und 1998). Allen Konzepten gemeinsam ist die Herstellung künstlicher Träger mit geeigneten Sauerstoffbindungseigenschaften. die den Sauerstofftransport *in vivo* gewährleisten können. Diese Eigenschaften richten sich nach dem gewünschten Anwendungsgebiet des Trägers und orientieren sich zumeist an denen des menschlichen Blutes. Natives, extrazellulär gelöstes Hämoglobin ist als Sauerstoffträger nicht geeignet, da es intravasal in seine Untereinheiten zerfällt und diese auf Grund ihres niedrigen Molekulargewichtes schnell über die Niere ausgeschieden werden. Deshalb versucht man, die intravasale Verweildauer natürlicher oder gentechnologisch hergestellter Hämoglobine im Organismus zu verlängern. Verlängerungen der intravasalen Verweildauer ergeben sich durch
- Mikroverkapselung von Hämoglobinlösungen in Liposomen, sogenannte Hämosomen (Ogata Y. (1994): "Characteristics of Neo Red Cells, Their Function and Safety: In Vivo Studies", Artificial Cells, Blood Substitutes, and Immobilization Biotechnologies 22: 875 - 881);
- kovalente intramolekulare Verknüpfungen, d. h. eine Stabilisierung der Quartärstruktur der Hämoglobine, durch bifunktionelle Vernetzer (Farmer M. C., et al. (1995): "Preclinical Data and Clinical Trials with Diaspirin Cross-linked Hemoglobin", - Tsuchida E. (Ed.): Artificial Red Cells, John Wiley 1995: 177 - 185; Bakker J. C., et al. (1988): "Properties of Hemoglobin Interdimerically Cross-linked with NFPLP", Biomaterials, Artificial Cells, and Immobilization Biotechnologies 16: 635 - 636) oder durch gentechnische Gewinnung (Looker D. et al. (1992): "A Human Recombinant Haemoglobin Designed for Use as a Blood Substitute", Nature 356: 258 -260);
- kovalentes Anknüpfen von Makromolekülen an das Hämoglobin, beispielsweise Polysaccharide, Dextrane, Hydroxyethylstärke, Inulin oder künstliche wasserlösliche Makromoleküle wie Polyethylenglykole (Xue H., Wong J. T.-F. (1994): "Preparation of Conjugated Hemoglobins", - Abelson J. N., Simon M. I. (Ed.): Methods of Enzymology, Volume 231 B, Academic Press 1994: 308 - 322; Tam S. C., et al. (1978): "Blood Replacement in Dogs by Dextran-Hemoglobin", Canadian Journal of Biochemistry 56: 981 - 984; Patentschriften DE-A 30 26 398 (1981): "Modifiziertes Hämoglobin enthaltender Blutersatz"; EP-A 0 069 026 (1982): "Oxygen Carrier"; EP-A 0 206 448 (1986): "Hemoglobin Combined with a Poly (Alkylene Oxide)"; US 5,234,903 (1993): "Chemically Modified Hemoglobin as an Effective, Stable, Non-immunogenic Red Blood Cell Substitute" und US 5,312,808 (1994): "Fractionation of Polyalkylene Oxide-Conjugated Hemoglobin Solutions");
- intermolekulares Vernetzen (Polymerisieren) der Hämoglobine mit bifunktionellen Vernetzern, (Gould S. A., et al. (1998): "The Clinical Development of Human Polymerized Hemoglobin", - Chang T. M. S. (Ed.): Blood Substitutes: Principles, Methods, Products and Clinical Trials, Volume 2, Karger Landes Systems 1998: 12 - 28; Pearce L. B., Gawryl M. S. (1998): "Overview of Preclinical and Clinical Efficacy of Biopure's HBOCs", - Chang T. M. S. (Ed.): Blood Substitutes: Principles, Methods, Products and Clinical Trials, Volume 2, Karger Landes Systems 1998: 82- 98; Bakker J. C., et al. (1992): "Preparation and Characterization of Crosslinked and Polymerized Hemoglobin Solutions", Biomaterials, Artificial Cells, and Immobilization Biotechnologies 20: 233 - 241).
Die letztgenannten künstlichen Sauerstoffträger auf der Basis vernetzter Hämoglobine besitzen gegenüber den anderen eine Reihe von Vorteilen: Ausreichend große vernetzte Hämoglobine (Hämoglobin-Polymere) verfügen über einen so geringen kolloid-osmotischen Druck, daß sie nicht nur - wegen ihres so geringen eigenen kolloid-osmotischen Druckes dann kombiniert mit einem Plasmaexpander - als Sauerstoff transportierendes Blutvolumensubstitut zum Ersatz fehlenden Blutes eingesetzt werden können, sondern insbesondere können sie auch - als Sauerstoff transportierendes Blutadditiv - in Blut addiert werden (Barnikol W. K. R., et al. (1996): "Hyperpolymere Hämoglobine als künstliche Sauerstoffträger. Ein innovativer Ansatz der medizinischen Entwicklung", Therapiewoche 46: 811 - 815). Indikationsgebiete für solche Sauerstofftransport-Additive sind die Behandlung vieler chronischer Sauerstoffmangelzustände, wie beispielsweise Anämien, Schlaganfälle oder Herzinfarkte. Die Behandlung mit einem solchen Additiv ist immer auch ohne einen Blutverlust möglich, dagegen sind sämtliche genannten anderen Sauerstoff transportierenden Volumensubstitute ausschließlich zur Behandlung akuter Sauerstoffmangelzustände nach Blutverlusten geeignet. Vernetzte Hämoglobine mit hohem Vernetzungsgrad besitzen darüber hinaus den Vorteil besonders langer intravasaler Verweildauer. Weiters muß nach ihrer Gabe nicht mit Blutdruckerhöhungen gerechnet werden, da sie auf Grund ihrer Größe die Blutgefäße nicht verlassen und somit nicht als Konstriktoren der Gefäßmuskulatur wirken können (s. w. u.).
Im Zentrum aller Entwicklungen steht, gleichwertig neben der Wirksamkeit, immer auch die Evaluation und gegebenenfalls Verbesserung der Unbedenklichkeit der künstlichen Sauerstoffträger (Fratantoni J. C. (1991): "Points to Consider in the Safety Evaluation of Hemoglobin-based Oxygen Carriers", Transfusion 31: 369 - 371). Beispielsweise wird in narkotisierten Ratten nach intravasaler Applikation intramolekular vernetzter Hämoglobine eine Blutdruckzunahme und ein Anstieg des Totalen Peripheren Gefäßwiderstandes beobachtet (Sharma A. C., et al (1993): "Role of NO Mechanism in Cardiovascular Effects of Diaspirin Cross-linked Hemoglobin in Anesthetized Rats", American Journal of Physiology 269: H 1379 - H 1388). Diese vasokonstriktorische Wirkung molekular disperser künstlicher Sauerstoffträger kann sowohl auf einer Hyperoxygenierung des Gewebes durch die sehr effektiven Träger beruhen (Rohlfs R. J., et al. (1998): "Arterial Blood Pressure Responses to Cell-free Hemoglobin Solutions and the Reaction with Nitric Oxide", Journal of Biological Chemistry 273: 12128 - 12134), oder auch auf die Inhibierung der lokalen Wirkung des Stickstoffmonoxids (NO) durch Hämoglobine zurückgeführt werden (Sharma A. C., et al. (1993) - siehe oben), denn Stickstoffmonoxid wirkt auf die glatte Muskulatur der Gefäßwände dilatierend (Rodeberg D. A., et al. (1995): "Nitric Oxide: An Overview", American Journal of Surgery 170: 292 - 303). Hämoglobin verläßt auf Grund seines niedrigen Molekulargewichtes das Gefäßsystem, bindet subendothelial abgegebenes Stickstoffmonoxid und verschiebt damit das an der Gefäßmuskulatur herrschende Gleichgewicht zwischen Vasodilatation und Vasokonstriktion zu letzterer. Vernetzen von Hämoglobinmolekülen (zu Multimeren: Oligomere und Polymere) verhindert die Diffusion aus den Gefäßen und damit die Blutdruckerhöhung durch die künstlichen Träger (Vogel W. M., Valeri C. R. (1986): "Coronary Constrictor Effect of Stroma-free Hemoglobin Solutions", American Journal of Physiology 251: H 413 - H 420).
Weiters ist der Einfluss der künstlichen Träger auf das Gerinnungssystem, das Abwehrsystem, insbesondere die Komplementaktivierung, die Aktivierung des Retikuloendothelialen Systems und die spezifische Immunantwort von großem Interesse. Es konnte gezeigt werden (Ning J., Chang T. M. S. (1990): "Effects of Homologous and Heterologous Stroma-free Hemoglobin and Polyhemoglobin on Complement Activation, Leukocytes and Platelets", Biomaterials, Artificial Cells, and Artificial Organs 18: 219 - 233; Feola M., Simoni J. (1991): "Biocompatibility of Hemoglobin Solutions: The Effects of Contaminants, Hemoglobin and Hemoglobin Derivates", Biomaterials, Artificial Cells, and Artificial Organs 19: 382), dass eine Komplementaktivierung nicht durch natives oder verknüpftes Hämoglobin selbst, sondern im wesentlichen durch Verunreinigungen, beispielsweise Endotoxine oder stromale Bestandteile der das native Hämoglobin zunächst enthaltenden Erythrozyten, ausgelöst wird. Somit kann der Einsatz hochreiner Ausgangslösungen zur Herstellung künstlicher Sauerstoffträger eine spätere Komplementaktivierung vermeiden.
Homologe Hämoglobine (Hämoglobine von den selben Tierspezies, denen es verabreicht wird), ob nativ oder vernetzt, wirken im Tiermodell auch nach wiederholter Gabe nicht immunogen, heterologe Hämoglobine (Hämoglobine von anderen als den Empfänger-Spezies) oder deren Vernetzungsprodukte können dagegen nach wiederholter Gabe eine Antikörperproduktion und anaphylaktische Reaktionen hervorrufen (Chang T. M. S. (1997): "How Safe are Modified Hemoglobins?", Blood Substitutes: Principles, Methods, Products and Clinical Trials, Volume 1, Karger Landes Systems 1997: 49 - 72). Verfahren zur Maskierung der Antigenität von Hämoglobinen sind die Mikroverkapselung oder die Konjugation mit Polyethylenglykol (z. B. Patentschrift US 4,179,337: "Non-immunogenic Polypeptides").
Im Rahmen der Untersuchung der Unbedenklichkeit künstlicher Sauerstoffträger wurde direkten Wechselwirkungen dieser mit Plasmaproteinen, mit denen sie bei intravasaler Anwendung in Kontakt kommen, bisher keine Beachtung geschenkt. In eigenen Untersuchungen konnte mit Hilfe eines neuen *in vitro -* Biokompatibilitätstests nachgewiesen werden, daß vemetzte Hämoglobine und Plasmaproteine in Abhängigkeit vom pH-Wert unterschiedlich verträglich sind. Es kommt zu Fällungen meist roter oder rötlich tingierter heller Präzipitate oder Niederschläge, die zumeist visuell erkenntlich sind - weniger offensichtliche Fällungen können durch empfindliche Trübungsmessungen erfaßt werden. Diese Fällungen betreffen entweder überwiegend die vernetzten Hämoglobine oder die Plasmaproteine, oder gleichermaßen beide. Der allfällige Nachweis der Beteiligung der Plasmaproteine, also das Vorliegen von Wechselwirkungen zwischen vernetzten Hämoglobinen und Plasmaproteinen, ist gegeben, weil im Plasmaüberstand, z. B. elektrophoretisch, auch eine Abnahme des Gehaltes plasmatischer Proteine, insbesondere der γ-Globuline, gemessen werden kann. Das Ausmaß der Hämoglobinfällungen ist dabei u. a. abhängig vom Molekulargewicht der vernetzten Hämoglobine, vom verwendeten Hämoglobin und bifunktionellen Vernetzer, sowie dem Vorhandensein kovalent gebundener Effektoren der Sauerstoff-Bindungseigenschaften der Hämgolobine (Domack U. (1997): "Entwicklung und in vivo-Evaluation eines künstlichen Sauerstoffträgers auf Basis von Rinderhämoglobin." Dissertation, Fachbereich Chemie, Johannes Gutenberg-Universität, Mainz 1997). Beispielsweise fallen von einem mit dem bifunktionellen Vernetzer Glutardialdehyd hergestellten, vernetzten Rinderhämoglobin mit einem mittleren Vernetzungsgrad um etwa 10 in Humanplasma bei pH-Werten kleiner als 7,5 größere Anteile aus. Die Modifikation der Sauerstoffbindungseigenschaften vernetzten Rinderhämoglobins durch kovalentes Anknüpfen von Pyridoxal-5'-Phosphat bewirkt eine weitere Herabsetzung der Plasmaverträglichkeit. In diesem Falle werden schon bei pH-Werten unter 8,0 Fällungen des Hämoglobins beobachtet. Mit Glutardialdehyd als Vernetzer hergestellt sind mit Pyridoxal-5'-Phosphat modifizierte, vernetzte Rinderhämoglobine nur bis zu einem Polymerisationsgrad von etwa fünf (Hämoglobin-Oligomere) im physiologisch relevanten pH-Bereich mit Humanplasma verträglich.
Ein positiver Einfluß auf die Bioverträglichkeit zwischen vernetztem Rinderhämoglobin und Humanplasma kann dagegen durch die Verwendung bifunktioneller Vernetzer erreicht werden, die bei der Polymerisation zusätzliche solvatisierende Gruppen einbringen. Beispielsweise sind mit dem Vernetzer 2,5-Diisothiocyanatobenzolsulfonsäure Rinderhämoglobin-Polymere herstellbar, die auch bei einem mittleren Polymerisationsgrad von 24 im Plasma ohne jegliche Fällungen löslich sind. Diese Polymere sind aber für eine Anwendung als künstliche Sauerstoffträger auf Grund ihrer Sauerstoff-Bindungseigenschaften völlig ungeeignet (Domack U. (1997), siehe oben).

Nach dem jetzigen Stand der Technik können mit vielen bifunktionellen Vernetzern keine vernetzten Hämoglobin insbesondere höherer Vernetzungsgrade hergestellt werden, ohne dass nach Mischen mit Plasma unter gewissen Bedingungen mit Unverträglichkeiten in Form von Ausfällungen gerechnet werden muss. Bei physiologischen und pathopysiologischen pH-Werten werden *in vitro* Unverträglichkeiten zwischen vernetzten Hämoglobinen und Plasmaproteinen beobachtet: Sowohl Plasmaproteine als auch vernetzte Hämoglobine fallen aus, in bestimmten Fällen kann die Fällung des einen oder des anderen überwiegen. Dies gilt insbesondere für stark vernetzte Hämoglobine (Hämoglobin-Polymere), weniger für geringer vernetzte Hämoglobine-Oligomere. Es ist zu erwarten, daß solche Hämoglobin-Plasmaprotein-Unverträglichkeiten bei Anwendungen *in vivo* auch im Gefäßsystem auftreten und in extremen Fällen zu multiplen Verschlüssen kleiner Gefäße führen.
Um die Funktionstüchtigkeit der Träger zu gewährleisten und schwerwiegende Nebenwirkungen bei der Anwendung im menschlichen oder tierischen Organismus, beispielsweise einen Schock durch kapilläre Stase, zu vermeiden, müssen die vernetzten Hämoglobine mit dem Blut, insbesondere mit den im Plasma enthaltenen Proteinen, verträglich sein, und zwar sowohl unter physiologischen, als auch unter allen möglichen pathophysiologischen Bedingungen: Dies gilt speziell auch für eine Azidose, wie sie lokal in mit Sauerstoff minder versorgtem Gewebe entsteht. Es müssen solche Wechselwirkungen vernetzter Hämoglobine mit Plasmabestandteilen unter allen im Organismus möglichen Bedingungen sicher verhindert werden, die zu einer Ausfällung einer Komponente führen.
In TH, Bd. 55,1999, Seiten 2147-2156 sowie Art Cell Blood subs, and Immmob, Biotech, Bd.22, Nr. 3,1994, Seite 923-931 berichten Ton T. Hai et al über die kovalente Anbindung von mit Diaspririn intramolekular verknüpften Hämoglobinen mit verbesserter Stabilität und Verweildauer.
In der EP 0 206 448 A1 werden Hämoglobine beschrieben, welche durch Anknüpfung von Polyalkylenoxid über Carboxygruppen eine besondere Stabilität bzw. Verweildauer erlangen.
Die WO A 91/07190 betrifft mit einem Polyethylenglykol konjugierte Hämoglobine mit verbesserten Sauerstoffbindungseigenschaften und besserer Stabilität.

Der Erfindung liegt daher die Aufgabe zugrunde, vemetzte Hämoglobine zu erzeugen, für die gewährleistet ist, dass sie nach einer intravasalen Applikation im Organismus mit den Plasmaproteinen - auch unter extremen pathophysiologischen pH-Werten -verträglich sind.
Die Aufgabe wird erfindungsgemäss gelöst, indem an die mit einem Vernetzer vernetzten Hämoglobinmoleküle Polyalkylenoxide mäßig hohen Molekulargewichtes, welche ein Verknüpfungsprodukt des Polyalkylenoxids mit einem eine terminale Hydroxygruppe des Polyalkylenoxid maskierenden Molekül sind, kovalent gebunden werden. In einem genügenden Ausmaß kovalent an die vernetzten Hämoglobine geknüpfte Polyalkylenoxide alleine bewirken, dass selbst unter extremen pathophysiologischen pH-Wert-Bedingungen (pH-Werte zwischen 6,8 und 7,4) *in vitro* keine Unverträglichkeiten von vernetzten Hämoglobinen und Plasmaproteinen in Form von Ausfallen der einen und/oder anderen Komponente feststellbar sind. Dies konnte nicht erwartet werden, da das Problem völlig neu ist und Lösungen ähnlicher oder auch nur analoger Probleme nicht verfügbar sind. Die bekannten Wirkungen einer Anknüpfung von Polyalkylenoxiden an Proteine sind, wie bereits erwähnt, gänzlich andere, als die hier beschriebenen. Darüber hinaus erscheint einerseits eine Kombination sowohl der Vernetzung des Hämoglobins als auch der kovalenten Anknüpfung von Polyalkylenoxid vordergründig als überflüssig: Beide Verfahren bewirken gemäß dem Stand der Technik das selbe, nämlich eine gewisse Verlängerung der intravasalen Verweildauer künstlicher Sauerstoffträger aus modifiziertem Hämoglobin, aber das notwendige Ausmaß wird bereits durch die Durchführung eines der Verfahren erreicht. Andererseits ist bekannt, dass Polyalkylene durch Lösungsmittelexklusion effektive Fältungsmittel für Proteine sind. Darauf beruhen technische Verfahren zur präparativen Trennung von Proteinen durch fraktionierte Fällung während konsekutiver Erhöhung der Konzentrationen der Polyalkylenoxide. Umso überraschender war es, dass bei der efindungsgemäßen Vorgehensweise das der Anmeldung zugrunde liegende Problem gelöst werden konnte.

Als Hämoglobin-Ausgangsmaterial für die erfindungsgemäße Lehre eignet sich monomeres, natives oder mit gewissen Effektoren, z. B. der Sauerstoffaffinität des Hämoglobins wie beispielsweise Pyridoxal-5'-Phosphat oder 2-Nor-2-Formyl-Pyridoxal-5'-Phosphat, (wie in Kothe et al. (1985), Surgery, Gynecology & Obstetrics 161: 563 -569 oder Van Der Plas et al. (1987), Transfusion 27: 425 - 430 und (1988), Transfusion 28: 525 - 530, beschrieben, weitere Zitate in: Rudolph A.S. et al. (Hrsg.): Red Blood Cell Substitutes: Basic Principles and Clinical Applications, Marcel Dekker, New York u.a. 1998; Tsuchida E. (Hrsg.): Blood Substitutes: Present and Future Perspectives, Elsevier Science, Amsterdam 1998, Volume 1 und Volume 2, Karger Landes, Basel u.a. 1997 und 1998, vgl. auch EP 0 528 841, dort wird die Pyridoxylierung von Hämoglobin beschrieben), chemisch umgesetztes und modifiziertes Hämoglobin vom Menschen, vom Schwein, oder vom Rind. Bevorzugt ist humanes und insbesondere Schweine-Hämoglobin. Das Hämoglobin kann gegebenenfalls auf bekannte Weise desoxygeniert (gegebenenfalls auch karbonyliert) werden.

Vernetzungen monomeren, nativen oder mit Effektoren verknüpften Hämoglobins mit etlichen Vernetzern sind bekannt und in der Literatur vielfach beschrieben, beispielhaft seien angeführt:
Die Patentschriften US 4,001,200 und US 4,001,401 betreffen vemetzte Hämoglobine sowie ihren Einsatz als Blutersatz und Plasmaexpander. Die Molekulargewichte (Molaren Massen) dieser vernetzten Hämoglobine betragen von 65 000 bis 1 000 000 g/mol. Sie können mittels einer Vielzahl genannter verknüpfender Agenzien hergestellt werden, wie beispielsweise Divinylsulfon, Epichlorhydrin, Butadiendiepoxid, Hexamethylendiisocyanat, den Dialdehyden Glyoxal und Glutardialdehyd sowie den Diimidoestern Dimethylsuberimidat, Dimethylmalonimidat und Dimethyladipimidat.
Die Patentschrift DE 24 49 885 betrifft (u. a.) auch vernetzte Hämoglobine, die durch Umsetzung nicht-vemetzter Hämoglobine mit etlichen Dialdehyden, beispielsweise Malondialdehyd, Succindialdehyd, Glutardialdehyd, Adipindialdehyd und Suberdialdehyd hergestellt werden können.
Die Patentschrift US 4,857,636 beschreibt die Herstellung verschiedener vernetzter Hämoglobine durch Umsetzung von Hämoglobin mit etlichen Dialdehyden und Polyaldehyden, beispielsweise einfachen wie Glutardialdehyd und Glyoxal, aber auch mit strukturell komplexeren, die durch oxidative Ringöffnung der zyklischen Halbazetal- und Halbketalstrukturen der Zuckermoleküle in Monosacchariden und Oligosacchariden sowie Derivaten dieser entstehen.
Die Patentschrift US 5,439,882 handelt von vernetzten Hämoglobinen, die durch Umsetzung mit den Dialdehyden o-Adenosin und o-ATP, entstanden durch ringöffnende Oxidation der Ribose in Adenosin und in Adenosintriphosphat, hergestellt sind. Diese vernetzten Hämoglobine besitzen Molekulargewichte von 65000 bis 390000 g/mol.
Die Patentschrift EP 0 201 618 betrifft ein Verfahren, aus hoch konzentrierten Lösungen monomerer Hämoglobine extrem hochmolekulare lösliche Hämoglobin-polymere, sogenannte Hyperpotymere (Molekulargewicht bis 15 000 000 g/mol), herzustellen.

Erfindungsgemäß werden bifunktionelle Vernetzer zur Vernetzung der Hämoglobine gewählt, nämlich Butandiepoxid, Divinylsulfon, ein Diisocyanat, insbesondere Hexamethylendiisocyanat. Zyklohexyldiisocyanat und 2,5-Bisisocyanatobenzolsulfonsäure, ein Di-N-Hydroxysuccinimidylester, ein Diimidoester, oder ein Dialdehyd, insbesondere Glyoxal, der analog reagierende Glykolaldehyd, oder Glutardialdehyd. Bevorzugt ist Glutardialdehyd.

Ganz besonders bevorzugt erfolgt die Vemetzung mit Glutardialdehyd, wie z. B. in Pötzschke H. und Bamikol W. (1992), Biomaterials, Artificial Cells, and Immobilization Biotechnology 20: 287 - 291, oder wie in den nachfolgenden Beispielen beschrieben.

Jeweils bezogen auf monomeres Hämoglobin werden molare Verhältnisse der verwendeten bifunktionellen Vernetzter - von 3- bis 60-fach, bevorzugt 6- bis 35-fach, eingesetzt. Bezüglich Glutardialdehyd wird bevorzugt zwischen einem 7- und 10-flachen molaren Überschuss am Glutardialdehyd eingesetzt. Chemisch nicht stabile Verknüpfungen, insbesondere die Schiffschen Basen, die bei der Reaktion von funktionellen Aldehydgruppen mit Aminogruppen der Hämoglobine entstehen, werden in bekannter Weise reduktiv durch Reaktion mit geeigneten Reduktionsmitteln, wie z. B. Natriumborhydrid, in einem hinreichenden molaren Überschuss, bezogen jeweils auf monomeres Hämoglobin, bevorzugt 2- bis 100-fach, insbesondere bevorzugt 5- bis 20-fach, unter geeigneten bekannten Bedingungen stabilisiert.

Kovalente Anknüpfungen von Polyalkylenoxiden an Proteine, insbesondere auch an (unvemetztes) Hämoglobin, sind etliche bekannt und in der Literatur beschrieben (den Stand der Technik beschreibt umfassend: Harris J. M. (Hrsg.): Poly (Ethylene Glyco/) Chemistry: Biotechnical and Biomedical Applications, Plenum, New York u. a. 1992). Bei sehr vielen dieser Verfahren erfolgt die Anknüpfung des Polyalkylenoxids über eine molekulare Brücke *("spacer"),* die beispielsweise ein bifunktioneller Verknüpfer schafft. Streng betrachtet wird in diesen Fällen also ein Verknüpfungsprodukt eines Polyalkylenoxids mit einem Verknüpfungsreagenz an das Protein geknüpft.

Zur kovalenten Anknüpfung der Polyalkylenoxide werden bevorzugt solche Derivate der Polyalkylenoxide verwendet, die ein verknüpfendes Agens mit einer funktionellen Gruppe bereits kovalent gebunden enthalten, welche eine direkte chemische Reaktion mit Amino-, Alkohol-, oder Sulfhydryl-Gruppen der Hämoglobine unter Bildung kovalenter Anknüpfungen der Polyalkylenoxide ergeben - beispielsweise Polyalkylenoxide mit reaktiven N-Hydroxysuccinimidylester-, Epoxid- (Glycidylether-), Aldehyd-, Isocyanat-, Vinylsulfon-, Jodazetamid-, Imidazolylformat-, Tresylatgruppen, u. a. Viele solche monofunktionell aktivierte Polyethylenglykole sind kommerziell erhältlich, z. B. die genannten, und zwar mit Molekulargewichten zwischen etwa 500 und 5 000 g/mol. Bevorzugt werden erfindungsgemäß Derivate eines Polyalkylenoxids, insbesondere ausgewählt aus Polyethylenoxid, Polypropylenoxid oder Kopolymeren hiervon, eingesetzt. Besonders bevorzugt sind Verknüpfungsprodukte des Polyalkylenoxids, insbesondere der genannten, mit einem eine terminale Hydroxygruppe maskierenden Molekül, insbesondere einem Ether, Ester, Esteramid mit kurzkettigen (C₁ - C₅) aliphatischen organischen Rest eingesetzt. Alternativ können nicht-aktive Polyalkylenoxide in jeder weiteren geeigneten Weise zunächst chemisch aktiviert oder, eventuell nach einer zusätzlich notwendigen Derivatisierung, durch chemische Verknüpfungsagenzien mit dem Hämoglobin verknüpft werden, beispielsweise mittels chemischer Reaktion mit Bromcyan, einem Karbodiimid wie beispielsweise 1-Ethyl-3-(3-Dimethylaminopropyl)karbodiimid oder N,N'-Dizyklohexylkarbodiimid, Cyanurchlorid (mit diesem aktivierte Polyethylenglykole, 4,6-Dichlor-s-triazin-Polyethylenglykole, sind ebenfalls kommerziell erhältlich), oder anderen, bekannten Verknüpfungsagenzien wie beispielsweise 2,2'-Dichlorbenzidin, p,p'-Difluor-m,m'-dinitrodiphenylsulfon, 2,4-Dichlornitrobenzol und weiteren (Überblick in: Harris J. M. (Hrsg.): Poly (Ethylene Glycol) Chemistry: Biotechnical and Biomedical Applications, Plenum, New York u. a. 1992). Als Polyalkylenoxide eignen sich besonders Polyethylenoxide (Polyethylenglykole), Polypropylenoxide (Polypropylenglykole), sowie Kopolymere (Mischpolymere) aus Ethylenoxid und Propylenoxid,insbesondere, wie erwähnt, gewisse Derivate dieser, wie eine Hydroxygruppe maskierende Verbindungen, beispielsweise (Mono-) Ether mit einem kurzkettigen Alkohol, vorzugsweise mit 1 bis 5 Kohlenstoffatomen, wie Monomethylether, Monoethylether, Monopropylether, u. s. w., (Mono-) Ester mit kurzkettigen Karbonsäuren, vorzugsweise mit 1 bis 5 Kohlenstoffatomen, wie Monomethylester, Monomethylester, Monopropylester, u. s. w. und Dehydratisierungsprodukte mit einem aliphatischen Amin mit 1 bis 5 Kohlenstoffatomen, wie Monomethylamin, Monoethylamin, Monopropylamin, u. s. w. mit dem oben angegebenen. Besonders bevorzugt sind Polyethylenglykole und deren genannte Derivate.
Das Molekulargewicht der verwendeten Polyalkylenoxide beträgt zwischen 200 und 5000 g/mol, insbesondere zwischen 500 und 2000 g/mol.
Diese werden vorzugsweise in einer Menge von 1 bis 40, insbesondere 4 bis 15 mol pro Mol Hämoglobin eingesetzt.

Wie bereits erwähnt ist die Anbindung von Polyalkylenoxiden an Proteine (z. B.: Patent US 4,179,337 (1979): "Non-immunogenic Polypeptides"), speziell auch an Hämoglobine, namentlich auch an künstliche Sauerstoffträger auf der Basis modifizierter Hämoglobine, bekannt (Patentschriften US 5,478,805 (1995): "Fradionation of Polyalkylene Oxide-Conjugated Hemoglobin Solution", US 5,386,014 (1995): "Chemically Modified Hemoglobin as an Effective, Stable, Non-immunogenic Red Blood Cell Substitute", EP-A 0 206 448 (1986): "Hemoglobin Combined with a Poly (Alkylene Oxide)". EP-A 0 067 029 (1982): "Oxygen Carrier"). Der Inhalt dieser Schriften ist daher vorliegend inkorporiert. Die Anknüpfung von Polyalkylenoxiden an künstliche Sauerstoffträger auf der Basis modifizierter Hämoglobine wurde nach der bekannten Literatur allerdings nie an einem vernetzten Hämoglobin vorgenommen, und diente immer dem Erreichen gänzlich anders gearteter Ziele, beispielsweise einer Verlängerung der intravasalen Verweildauer, oder auch zur Verminderung der immunogenen Potenz der künstlichen Sauerstoffträger.

Die Durchführung der erfindungsgemäßen Anknüpfungen ist analog wie oben beschrieben und richtet sich nach den Erfordernissen der gewählten chemischen Reaktionen: Native und modifizierte, monomere und vemetzte Hämoglobine sind Polyelektrolyte und befinden sich deshalb zur Reaktion mit aktiven Polyalkylenoxiden oder zur verknüpfenden Umsetzung mit Polyalkylenoxiden mittels Aktivatoren oder Verknüpfungsagenzien in wässrigen Elektrolyten mit Ionenkonzentrationen bis 300 mmol/L, vorzugsweise zwischen 50 und 170 mmol/L. Die Temperaturen während der Reaktionen betragen zwischen 2 und 65 °C, bevorzugt zwischen 3 und 30 °C, die Protonenaktivität in den Lösungen, ausgedrückt als pH-Werte, zwischen 5 und 11, bevorzugt zwischen 6,0 und 10,5 und die Reaktionszeiten, je nach der gewählten speziellen Reaktion zur Anknüpfung der jeweiligen Polyalkylenoxide an die entsprechenden Hämoglobine, auch in Abhängigkeit von Temperatur, pH-Wert, Ionenkonzentration u. s. w., zwischen wenigen Minuten und bis zu 24 Stunden, bevorzugt weniger als 5 Stunden und ganz bevorzugt weniger als 2 Stunden.

Das vemetzte Hämoglobin kann somit mit Hilfe der bekannten Methoden mit Polyalkylenoxid verknüpft werden, wie oben beschrieben, beispielsweise durch direkte Kombination mit Hilfe eines Kondensationsmittels, wie Bromcyan, oder mit Hilfe eines Vemetzungsreagenz, wie beispielsweise Cyanurchlorid (vgl. DE-OS 30 26 398), oder durch Reaktion mit einem aktivierten Polyalkylenoxid, beispielsweise einem N-Hydroxysuccinimidylester eines

Polyalkylenoxidderivates. Auf diese Weise werden mindestens 1, insbesondere 1 bis 40 und vorzugsweise 4 bis 15 Moleküle des erfindungsgemäß verwendeten Polyalkylenoxids je Molekül monomeren Hämoglobins verknüpft.

Beispielhaft können folgende Verfahren für die Anknüpfung der Polyalkylenoxide angewendet werden, wobei deren strukturelle Integrität erhalten bleibt
(1) (Nicht aktiviertes) Polyethylenglykol wird mit der 2- bis 5-fachen molaren Menge, vorzugsweise der 3-fachen molaren Menge an Bromcyan bei einem pH-Wert von 9 bis 10 umgesetzt. Das restliche Bromcyan wird durch Gelfiltration, Dialyse etc. aus dem Reaktionsgemisch entfernt und das Produkt wird dann mit einer erforderlichen, z. B. 0,1- bis 0,002-fachen, vorzugsweise der 0,02- bis 0,01-fachen molaren Menge an Hämoglobin bei pH 7 bis 9, vorzugsweise 7,5 bis 8, in wässriger Lösung umgesetzt (vgl. DE-OS 3 026 398);
(2) Polyethylenglykol wird in Benzol gegeben, welches eine überschüssige Menge an Natriumkarbonat enthält, und dann mit der 2- bis 5-fachen molaren Menge, vorzugsweise der 3- bis 4-fachen molaren Menge an Cyanursäurechlorid umgesetzt. Das Reaktionsprodukt, Polyethylglykol-4,6-dichlor-s-triazin, wird abgetrennt und mit der gewünschten Menge, z. B. 1 bis 0,002 mol, vorzugsweise 0,1 bis 0,01 mol, bezogen auf ein Mol des vorstehend genannten Reaktionsprodukts, an Hämoglobin in einer Pufferlösung mit einem pH-Wert von 8 bis 9,5 umgesetzt (vgl. DE-OS 30 26 398);
(3) Aktiviertes Polyalkylenoxid, beispielsweise ein N-Hydroxysuccinimidylester eines Polyalkylenoxids, wird in einem 1- bis 40-fachen molaren Überschuss bezogen auf monomeres Hämoglobin zu einer wässrigen Lösung mit einem pH zwischen 7 und 10 eines mit dem Polyalkylenoxid zu verknüpfenden Hämoglobins gegeben und reagieren lassen.

Die vorstehend erläuterten Methoden lassen sich auch im Fall der anderen erfindungsgemäss verwendeten Polymeren anwenden.

Die chemische Anbindung der Polyalkylenoxide an die künstlichen Sauerstoffträger aus vernetzten Hämoglobinen erfolgt dann im Verlauf der Herstellung der erfindungsgemäßen Hämoglobin-Derivate, indem die Polyalkylenoxid-Derivate an das bereits synthetisierte vemetzte Hämoglobin angekoppelt werden, d.h. im Anschluss an die Umsetzung der hochreinen, nativen oder mit Effektoren modifizierten Hämoglobin-Monomere mit einem bifunktionellen Vernetzer.

Das erhaltene erfindungsgemäße Hämoglobin-Derivat kann auf bekannte, übliche Weisen gereinigt werden, z.B. durch Zentrifugation, Klärfiltration, Ultrafiltration oder eine präparative Chromatographie, z.B. Volumenausschlusschromatographie beispielsweise an Sephadex G-25 Gel oder wie in den obengenannten Druckschriften, oder EP-A 0 854 151, EP-A 95 107 280 oder in Curling J.M.: Methods of Plasma Protein Fractionation, Academic Press, London, 1980, beschrieben.

Erfindungsgemäß wird monomeres Hämoglobin, bevorzugt im desoxygenierten Zustand, in einem wässrigen Elektrolyten (der z. B. NaHCO₃ oder NaCl oder Natriumlaktat oder mehrere dieser enthält), zunächst vernetzt, beispielsweise mit bifunktionellen Vernetzern wie Butandiepoxid, Divinylsutfon, einem Diisocyanat, insbesondere Hexamethylendiisocyanat, Zyklohexyldiisocyanat und 2,5-Bisisocyanatobenzolsulfonsäure, einem Di-N-Hydroxysuccinimidylester, einem Diimidoester, oder einem Dialdehyd, insbesondere Glyoxal, dem analog reagierenden Glykolaldehyd, und ganz besonders bevorzugt Glutardialdehyd, z. B. in einem 3- bis 60-fachen, bevorzugt einem 6- bis 35-fachen molaren Überschuss bezogen auf monomeres Hämoglobin, insbesondere einem 7- bis 10-fachen molaren Überschuss im Falle des Glutardialdehyd. Überschuss-Reaktanden können auf übliche Weise durch geeinete Zusätze entfernt werden, z. B. durch Zusatz von Natriumcyanoborhydrid oder Natriumborhydrid im Falle der Dialdehyde (wie beispielsweise Glutardialdehyd) z. B. in einem 2- bis 100-fachen, insbesondere einem 5- bis 20-fachen molaren Überschuss, bezogen wiederum auf das monomere Hämoglobin.
Das erhaltene vemetzte Hämoglobin in Lösung kann sodann direkt mit einem der oben genannten Polyalkylenoxide, beispielsweise einem Polyethylenglykol, einem Polypropylenglykol, oder einem Kopolymerisat aus Ethylenoxid und Propylenoxid oder einem der obengenannten Derivate hiervon, insbesondere einem aktivierten Polyethylenglykol wie Methoxy-Polyethylenglykol-N-Hydroxysuccinimidylpropionat (mPEG-SPA), wie beschrieben verknüpft werden. Dazu wird das Polyalkylenoxid im Überschuss, z. B. im 1- bis 40-fachen, insbesondere vorzugsweise im 4- bis 15-fachen molaren Verhältnis bezogen auf monomeres Hämoglobin, eingesetzt. Der verbleibende Überschuss kann auf bekannte Weise wieder entfernt oder inaktiviert werden, z. B. durch Umsetzung mit überschüssigem Lysin. Die Polyalkylenoxide haben eine molare Masse von 200 bis 5 000, insbesondere 500 bis 2 000 g/mol.
Die so erhaltene Lösung kann dann auf geeignete bekannte Weise, z. B. chromatographisch (z. B. durch präparative Volumenausschluss-Chromatographie) durch Zentrifugation, (Klär-) Filtration oder Ultrafiltration, oder durch Fällung, z. B. mit Polyethylenoxid, gereinigt und nachfolgend zu einer pharmazeutischen Zubereitung weiterverarbeitet werden.

Die Elektrolytkonzentration und damit auch der pH-Wert kann jeweils entsprechend den erforderlichen Bedingungen wie beschrieben auf bekannte Weise eingestellt werden.

Auf diese Weise wird als Produkt ein vernetztes und mit Polyalkylenoxid verknüpftes Hämoglobin erhalten, welches mit Plasma auch unter extremen physiologischen Bedingungen insbesondere des pH-Wertes überraschenderweise völlig verträglich ist. Die Verträglichkeit ist dabei unabhängig von der Art und dem Molekulargewicht des Hämoglobins, vom verwendeten Vernetzer, Effektoren oder der Art des eingesetzten Polyalkylenoxids.

Dies konnte im Hinblick auf den Stand der Technik nicht erwartet werden, da dort sowohl durch kovalente Anknüpfung von Polyalkylenoxiden als auch durch Vemetzung *in vivo* lediglich eine verbesserte Verweilzeit bzw. eine geringere Immunogenität, jedoch keine Plasmaverträglichkeit, erzielt wurde.
Die überraschenden Vorteile des erfindungsgemäßen Hämoglobin-Derivates lassen sich wie folgt zusammenfassen:
1. Kovalent an vernetztes Hämoglobin angeknüpftes Polyalkylenoxid ergibt mit Plasma verträgliche künstliche Sauerstoffträger. Die Plasmaverträglichkeit dieser vernetzten Hämoglobine ist dabei nicht vom verwendeten Hämoglobin, von der Molekülgröße der vernetzten Hämoglobine, oder vom Vernetzer abhängig.
2. Durch die Anbindung der Polyalkylenoxide an die vernetzten Hämoglobine wird auch unter ungünstigen pH-Wert-Bedingungen sicher gestellt, daß im Organismus nicht mit Wechselwirkungen zwischen Plasmaproteinen und vernetzten Hämoglobinen gerechnet werden muß, die zu Fällungen der vernetzten Hämoglobine oder von Plasmaproteinen führen.
3. Die Modifikation vernetzten Hämoglobins mit Polyalkylenoxid erlaubt die intravasale Anwendung von vernetzten Hämoglobinen hoher Multimerisationsgrade (Hämoglobin-Polymere) - ohne eine solche Anbindung wäre nur eine Applikation von Oligomeren möglich, um Fällungserscheinungen oder anderweitige Wechselwirkungen beispielsweise mit Proteinen, aber auch mit den Blutzellen im Plasma zu vermeiden. Diesen Hämoglobin-Polymeren erschließen sich damit, neben der Anwendung als Substitut verlorenen Blutvoluminens, weitere Anwendungsgebiete aus dem Bereich chronischer Sauerstoffmangelzustände. Auf Grund ihrer hohen molekularen Größe können sie einem Patienten als zusätzlicher Sauerstoffträger - als ein Sauerstoff transportierendes Additiv - gegeben werden.
4. Zudem läßt die Anbindung von Polyalkylenoxiden eine erhöhte vaskuläre Verweildauer, wie auch eine verringerte Immunogenität erwarten.

Insofern können die erfindungsgemäßen Hämoglobin-Derivate als solche oder in Form geeigneter, z. B. pharmazeutischer Zubereitungen als künstliche Sauerstoffträger intravasal als Pharmazeutikum oder für biomedizinische Zwecke, als Ersatz des Blutes zur Behandlung eines Blutvolumenmangels, als Zusatz zum Blut zur Behandlung pathogener Sauerstoffmangelzustände, oder als eine Nährlösung, im menschlichen oder tierischen Organismus, in Organen oder in biotechnischen Anwendungen, verwendet werden. Zur Herstellung der zu verabreichenden Produkte werden die erfindungsgemäßen Hämoglobin-Derivate in geeigneten Medien, wie Infusionslösungen, beispielsweise in wässriger Kochsalz- oder Glukoselösung, vorzugsweise in dem Blutplasma isotonischen Konzentrationen, gelöst.

Besonders bevorzugte Ausgestaltungen der Erfindung werden nachfolgend zunächst anhand eines allgemeinen Herstellungsverfahrens der vernetzten und mit Polyalkylenoxid verknüpften Hämoglobine näher erläutert:
1. Gereinigtes Schweine-, Human- oder Rinderhämoglobin mit einer Konzentration zwischen 10 und 420 g/L, bevorzugt zwischen 150 und 400 g/L, ist in einer wässrigen Natriumhydrogenkarbonat-Lösung gelöst, diese besitzt eine Konzentration zwischen 10 und 150 mmol/L, bevorzugt zwischen 40 und 60 mmol/L. Durch Überströmen mit reinem Stickstoff und Rühren dieser Hämoglobinlösung erfolgt bei einer Temperatur aus dem Bereich von 2 bis 42 °C, bevorzugt zwischen 3 und 25 °C, eine Desoxygenierung des Hämoglobins. Der pH der Lösung wird mit Milchsäure oder Natronlauge (einer Konzentration zwischen 0,1 und 1 mol/L) auf einen Wert zwischen 6 und 9, bevorzugt zwischen 6,5 und 7,5 titriert.
2. Bei dem so eingestellten pH-Wert erfolgt dann die Reaktion des Hämoglobins mit einem bifunktionellen Vernetzer, ausgesucht aus Butandiepoxid, Divinylsulfon, einem Diisocyanat, insbesondere Hexamethylendiisocyanat, Zyklohexyldiisocyanat und 2,5-Bisisocyanatobenzolsulfonsäure, einem Di-N-Hydroxysuccinimidylester, einem Diimidoester, oder einem Dialdehyd, insbesondere Glyoxal, dem analog reagierenden Glykolaldehyd, und ganz besonders bevorzugt Glutardialdehyd. Das molare Verhältnis des Vernetzers zum monomeren Hämoglobin beträgt zwischen 3 und 60, bevorzugt zwischen 6 und 35. Nach Vemetzung mit einem der genannten Dialdehyde werden z. B. die entstandenen Schiffschen Basen mit Natriumborhydrid, in einem molaren Verhältnis zum monomeren Hämoglobin zwischen 2 und 100, bevorzugt zwischen 5 und 20, reduziert. Diese Reduktion erfolgt bei einem pH zwischen 7,5 und 9, bevorzugt zwischen 7,8 und 8,8; dieser pH-Wert wird, wie oben (Nr. 1) beschrieben, mit Natronlauge oder Milchsäure eingestellt.
3. Nach erneuter Einstellung der pH-Werte zwischen 7 und 9,5 (mit Natronlauge oder Milchsäure) werden die vernetzten Hämoglobine mit einem der o. g. Polyalkylenoxid-Derivate verknüpft, das in einem molaren Verhältnis zum monomeren Hämoglobin von 1 bis 40, bevorzugt zwischen 4 und 15, dem Reaktionsgemisch zugegeben wind. Die Molekulargewichte der verwendeten Polyalkylenoxide betragen zwischen 200 und 5 000 g/mol, bevorzugt zwischen 500 und 2 000 g/mol. Die Polyalkylenoxide können bereits, wie oben erwähnt, zur Reaktion insbesondere mit Aminogruppen der Hämoglobine monofunktionell aktiviert sein, oder, wie ebenfalls beschrieben, aktiviert oder passiv angeknüpft werden.

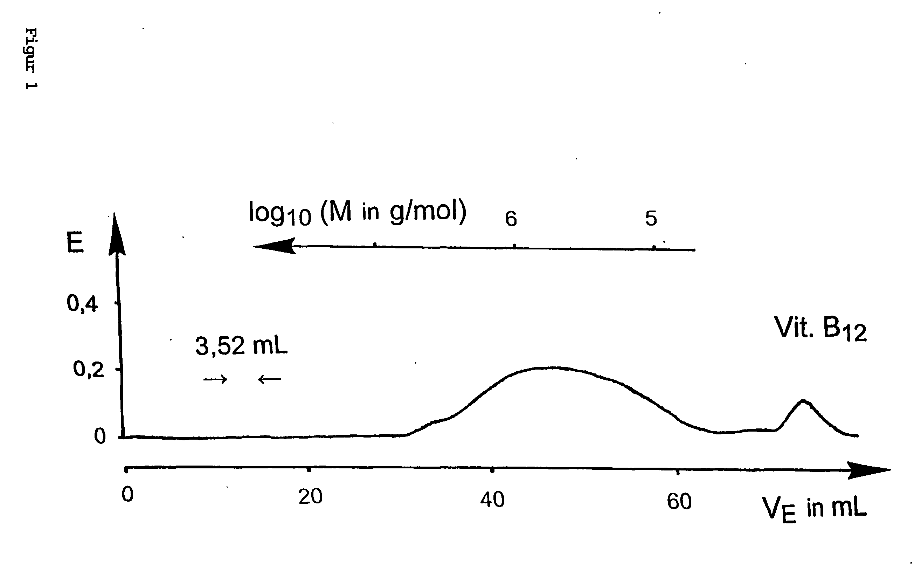

Die Erfindung wird anhand nachfolgender Beispiele näher erläutert. Hierbei zeigen die Figuren 1 bis 6 folgendes:
Figur 1:
   Eine massen-gewichtete Verteilung der Moleküfgrößen und Molekulargewichte (M) des Glutardialdehyd-Schweinehämoglobin-Polymeren aus Beispiel 1, dargestellt als Volumenausschluss-Chromatogramm (erhalten mit Sephacryl S-400 HR - Gel, Pharmacia, Freiburg, D). E₄₂₅ₙₘ ist die Extinktion im Chromatographie-Eluat bei 425 nm, V_{E} das Elutionsvolumen, Vitamin B₁₂ dient als Referenzsubstanz (innerer Standard).
Figur 2:
   Ergebnisse des *in vitro -* Verträglichkeitstests einer Mischung der Schweinehämoglobin-Polymeren aus Beispiel 1 mit humanem Plasma (isovolämische Mischung), dargestellt als Änderung der relativen Hämoglobinkonzentrationen in Abhängigkeit vom pH-Wert nach Ansäuerung mit Milchsäure ●: Schweinehämoglobin-Polymere (ohne kovalent angeknüpftes Polyalkylenoxid),
   ■: Schweinehämoglobin-Polymere mit kovalent gebundenem PEG-1000,
   ◆: Schweinehämoglobin-Polymere mit kovalent gebundenem PEG-2000 .
Figur 3:
   Eine massen-gewichtete Verteilung der Moleküfgrößen und Molekulargewichte (M) des fraktionierten Glutardialdehyd-Humanhämoglobin-Polymeren aus Beispiel 2, dargestellt als Volumenausschluss-Chromatogramm (erhalten mit Sephacryl S-400 HR - Gel, Pharmacia, Freiburg). E₄₂₅ₙₘ ist die Extinktion im Chromatographie-Eluat bei 425 nm, V_{E} das Elutionsvolumen, Vitamin B₁₂ dient als Referenzsubstanz (innerer Standard).
Figur 4:
   Ergebnisse des *in vitro -* Verträglichkeitstests einer Mischung der fraktionierten Humanhämoglobin-Polymeren aus Beispiel 2 mit humanem Plasma (isovolämische Mischung), dargestellt als Änderung der relativen Hämoglobinkonzentrationen in Abhängigkeit vom pH-Wert nach Ansäuerung mit Milchsäure
   ●: Humanhämoglobin-Polymere (ohne kovalent angeknüpftes Polyalkylenoxid),
   ■: Humanhämoglobin-Polymere mit kovalent gebundenem PEG-1000.
Figur 5:
   Eine massen-gewichtete Verteilung der Molekülgrößen und Molekulargewichte (M) des fraktionierten Glutardialdehyd-Rinderhämoglobin-Polymeren aus Beispiel 3, dargestellt als Volumenausschluss-Chromatogramm (erhalten mit Sephacryl S-400 HR - Gel, Pharmacia, Freiburg). E₄₂₅ₙₘ ist die Extinktion im Chromatographie-Eluat bei 425 nm, V_{E} das Elutionsvolumen, Vitamin B₁₂ dient als Referenzsubstanz (innerer Standard).
Figur 6:
   Ergebnisse des *in vitro -* Verträglichkeitstests einer Mischung der fraktionierten Rinderhämoglobin-Polymeren aus Beispiel 3 mit humanem Plasma (isovolämische Mischung), dargestellt als Änderung der relativen Hämoglobinkonzentrationen in Abhängigkeit vom pH-Wert nach Ansäuerung mit Milchsäure
   ●: Rinderhämoglobin-Polymere (ohne kovalent angeknüpftes Polyalkylenoxid),
   ■: Rinderhämoglobin-Polymere mit kovalent gebundenem PEG-1000.

### Beispiel 1

### Kovalente Anknüpfung monofunktionellen N-Hydroxy-Succinimidylpropionat-Polyethylenglykols (mPEG-SPA) mit Molaren Massen von 1000 g/mol (mPEG-SPA-1000) und 2000 g/mol (mPEG-SPA-2000) an kovalent vernetztes Schweinehämoglobin

Die Synthese der vernetzten Schweinehämoglobine erfolgte (leicht modifiziert) gemäß den Vorschriften aus Dinkelmann S. ("Präparation und in vitro Charakterisierung eines künstlichen Sauerstoffträgers auf der Basis von Schweinehämoglobin und seine Evaluierung im Kleintier", Dissertation, Fachbereich Medizin, Johannes Gutenberg-Universität, Mainz 1997, vgl. auch Pötzschke H. et al., Art. Cells, Blood Subst. and lmmob. Biotechn. 25 (1997), 527-540) und Domack U. ("Entwicklung und in vivo-Evaluation eines künstlichen Sauerstoffträgers auf Basis von Rinderhämoglobin", Dissertation, Fachbereich Chemie, Johannes Gutenberg-Universität Mainz 1997): Hochreines, konzentriertes, desoxygeniertes Schweinehämoglobin gelöst in einem wässrigen Elektrolyten der Zusammensetzung 50 mmol/L NaHCO₃ und 100 mmol/L NaCl wurde bei Raumtemperatur mit dem 14-fachen molaren Überschuss an Glutardialdehyd umgesetzt. Natriumcyanoborhydrid, im 10-fachen molaren Überschuss zum (monomeren) Hämoglobin zugesetzt, reduzierte die bei der Vemetzung entstandenen Schiffschen Basen und stabilisierte die kovalente Vernetzung. Die erhaltene Lösung der vernetzten Hämoglobine wurde in drei Teile (A, B und C) geteilt und unterschiedlich weiter verarbeitet.
Teil A blieb unverändert, die Bestimmung der Molekulargewichtsverteilung (gemäß Pötzschke H. et al. (1996): "Vernetzte globuläre Proteine - eine neue Klasse halbsynthetischer polymerer Moleküle: Charakterisierung ihrer Struktur in Lösung am Beispiel hyperpolymeren Hämoglobins und Myoglobins mittels Volumenausschluß-Chromatographie, Viskosimetrie, Osmometrie und Lichtstreuung", Macromolecular Chemistry and Physics 197, 1419 - 1437, sowie Pötzschke H. et al. (1996): "Ein neuartiges Verfahren zur Bestimmung Molarer Massen breit verteilter Polymerer mit Hilfe der Gel-Chromatographie und der Viskosimetrie am Beispiel Hämoglobin-Hyperpolymerer", Macromolecular Chemistry and Physics 197, 3229 - 3250) unter Anwendung der Volumenausschluss-Chromatographie mit dem Gel Sephacryl S-400 HR (Pharmacia Biotech, Freiburg, D) ergab für das vemetzte Schweinehämoglobin (Abbildung 1 zeigt ein Chromatogramm) einen Modalwert der Molekulargewichtsverteilung von 520 kg/mol.
Die Polymeren des Anteils B wurden mit monofunktionell aktivem mPEG-SPA-1000 (Shearwater Polymers Europe, Enschede, NL) kovalent verknüpft: Zunächst wurde Natriumhydrogencarbonat als Festsubstanz bis zu einer Endkonzentration von 150 mmol/L zur Lösung der vernetzten Hämoglobine addiert, anschließend erfolgte die Zugabe von mPEG-SPA-1000 im 12-fachen molaren Überschuss (bezogen auf die Hämoglobin-Monomeren) ebenfalls als Festsubstanz. Nach einer Reaktionszeit von einer Stunde wurde Lysin im 60-fachem molaren Überschuss (bezogen auf Hämoglobin) zugegeben und reagierte mit noch aktiven mPEG-SPA-1000-Molekülen. Teil C: Mit der Lösung der vernetzten Hämoglobine wurde genauso verfahren wie für Teil B beschrieben, jedoch unter Verwendung von mPEG-SPA-2000 (Shearwater Polymers Europe, Enschede, NL).
Anschließend erfolgte ein Lösungsmitteltausch in den drei Lösungen A, B und C (mit Hilfe einer Ultrafiltration, "Ultraminisette 10 kDa", Pall Gelman Sciences, Roßdorf, D, oder einer Volumenausschluss-Chromatographie am Gel "Sephadex G-15 M", Pharmacia Biotech, Freiburg, D) zu einer Lösung in einem wässrigen Elektrolyten (StLg) der Zusammensetzung: 125 mM NaCl, 4,5 mM KCI und 3 mM NaN₃.
Die Untersuchung der Plasmaverträglichkeit des nicht modifizierten (A), sowie des mit PEG modifizierten vernetzten Schweinehämoglobins (B und C) erfolgte mittels eines standardisierten in *vitro*-Fällungstests (Domack U. (1997), s.o.). Die Hämoglobinlösungen wurden mit gleichen Mengen frisch gewonnenen, steril filtrierten menschlichen Plasmas gemischt und anschließend zu jeweils 500 µL der Mischung bis zu 20 µL 0,5-molare Milchsäure zugesetzt und eingemischt, so dass sich für jedes zu untersuchende Hämoglobin-Derivat jeweils pH-Werte aus einem Bereich zwischen etwa 7,4 bis 6,8 ergaben. Nach einer Inkubation von 30 Minuten bei Raumtemperatur und Zentrifugation der Proben erfolgte die Bestimmung des Hämoglobingehaltes (modifizierte Cyanhämiglobin-Methode nach Drabkin: "Hämoglobin-Farbtest MRP 3", Boehrimger Mannheim, D) und des zugehörigen pH-Wertes (Blutgasanalysator "ABL 5", Radiometer, Willich, D) im Überstand.

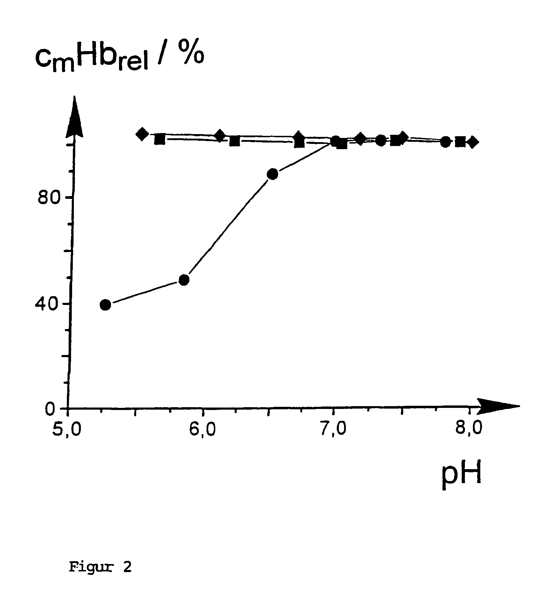

In Abbildung 2 sind die relativen Hämglobinkonzentrationen (bezogen auf die Ausgangs-Hämoglobinkonzentration vor Milchsäurezugabe) in Abhängigkeit vom pH-Wert der Hämoglobin-Plasma-Mischung dargestellt, Verminderungen ergeben sich durch Ausfällung nicht verträglicher Anteile. Für pH-Werte kleiner 7,0 wurden bei der Probe A (nicht modifiziertes Schweinehämoglobin-Polymer) rote Fällungen beobachtet, die sich als Abnahme der Hämoglobinkonzentration darstellen. Für dieses vernetzte Schweinehämoglobin ist im pH-Intervall von 7,4 bis 6,8 auch *in vivo* mit solchen Unverträglichkeiten zu rechnen. Dagegen waren bei den mit PEG modifizierten vernetzten Hämoglobinen B und C keine Fällungen im physiologisch interessanten Bereich zwischen den pH-Werten 7,4 und 6,8 und darüber hinaus noch bis 5,5 zu beobachten, der Hämoglobingehalt nahm nicht ab.
Im physiologisch und pathophysiologisch interessanten pH-Bereich von 7,4 bis 6,8 konnte somit durch das kovalente Anbinden sowohl von PEG-1000 als auch von PEG-2000 an vernetzte Schweinehämoglobine ein wirksamer Schutz dieser, als auch der Plasmaproteine vor durch Wechselwirkungen verursachten Fällungen erreicht werden.

### Beispiel 2

### Kovalentes Anknüpfen von mPEG-SPA-1000 (N-Hydroxy-Succinimidylpropionat-Polyethylenglykol mit einer Molaren Masse von 1000 g/mol) an vernetztes Humanhämoglobin

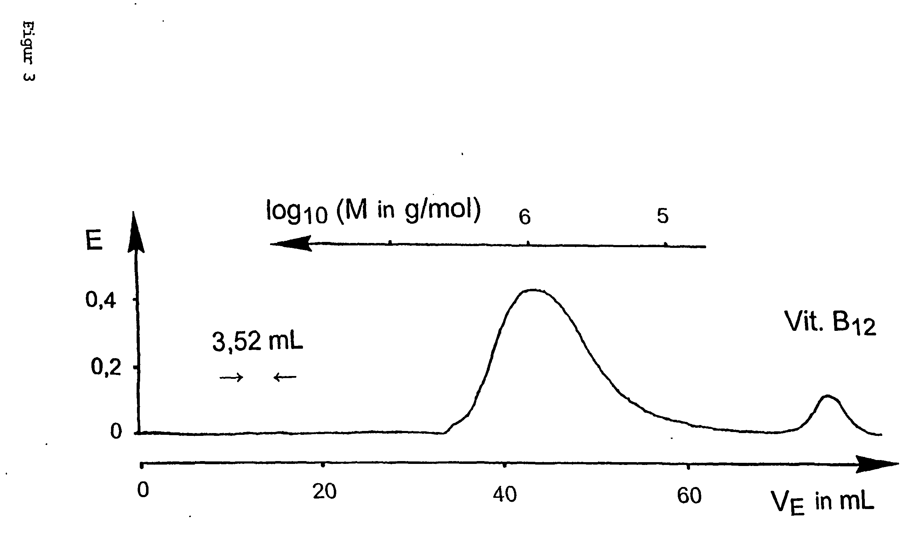

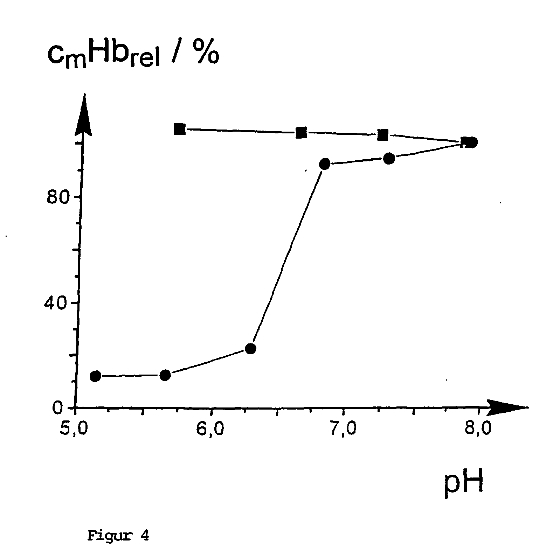

Die Synthese des mit Glutardialdehyd vernetzten Humanhämoglobins erfolgte wie in Beipiel 1, jedoch unter Verwendung von hochreinem, konzentrierten Humanhämoglobin und Einsatz des 16-fachen molaren Überschusses des Vernetzers. Polymere wurden durch Fraktionieren der Lösung der Vernetzungsprodukte mit Hilfe einer präparativen Volumenausschluss-Chromatographie (gemäß EP-A 95 10 72 80.0: "Verfahren zur Herstellung molekular-einheitlicher hyperpolymerer Hämoglobine" mit Sephacryl S-300 HR - Gel, Pharmacia Biotech, Freiburg, D) gewonnen (hier als die zuerst eluierten 57 Massen-% des vernetzten Hämoglobins).
Die vernetzten Hämoglobine wurden in zwei Teile A und B aufgeteilt. Das Hämoglobin A (vergleiche Abbildung 3) erwies sich als überwiegend polymeres Hämoglobin mit einem Modalwert der Molekulargewichtsverteilung von 950 kg/mol (vergleiche Beispiel 1). Kovalentes Anbinden von monofunktionell aktivem mPEG-SPA-1000 erfolgte analog der in Beispiel 1 für vernetztes Schweinehämoglobin beschriebenen Vorgehensweise□ Nach der Addition von Natriumhydrogenkarbonat (bis zu 150 mM) zur Lösung der Polymeren konnte ein 12-facher molarer Überschuss mPEG-SPA-1000 mit den Hämoglobin-Monomeren reagieren. Im Anschluß an eine Reaktionszeit von einer Stunde wurde Lysin im 60-fachen molaren Überschuss zum ,Abfangen' noch aktiver Moleküle des mPEG-SPA-1000 zugegeben. Als Vorbereitung zum *in-vitro* Biokompatibilitätstest folgte das Umwaschen (Lösungsmitteltausch) der Lösungen A und B in den wässrigen Elektrolyten "StLg" (ganz analog wie im Beispiel 1 beschrieben).
Der Fällungstest - die Ergebnisse sind in Abbildung 4 dargestellt - ergab für die vernetzten Hämoglobine A im gesamten untersuchten pH-Intervall von 7,9 bis 5,1 rote Fällungen. So fielen beispielsweise im Falle der Simulation einer Azidose mit einem pH-Wert von 6,8 ca. 8% der Hämoglobinpolymeren aus. Bei einem pH von 5,7 wird ein Maximum der Fällungen erreicht. Allein die Modifikation mit mPEG-1000 verhindert das Auftreten von Hämoglobin-Präzipitaten bis weit in den sauren Bereich, bis zu einem pH von 5,7.

### Beispiel 3

### Kovalentes Anknüpfen von mPEG-SPA-1000 an vernetztes Rinderhämoglobin

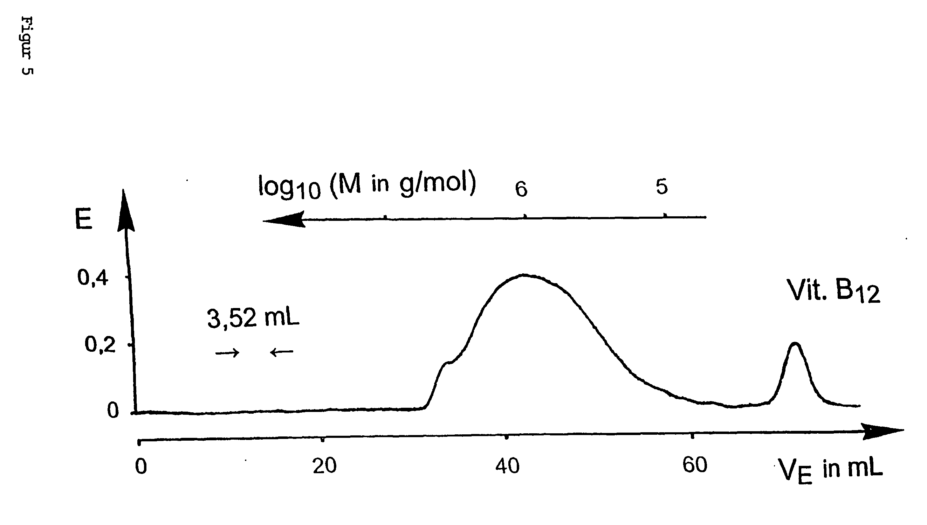

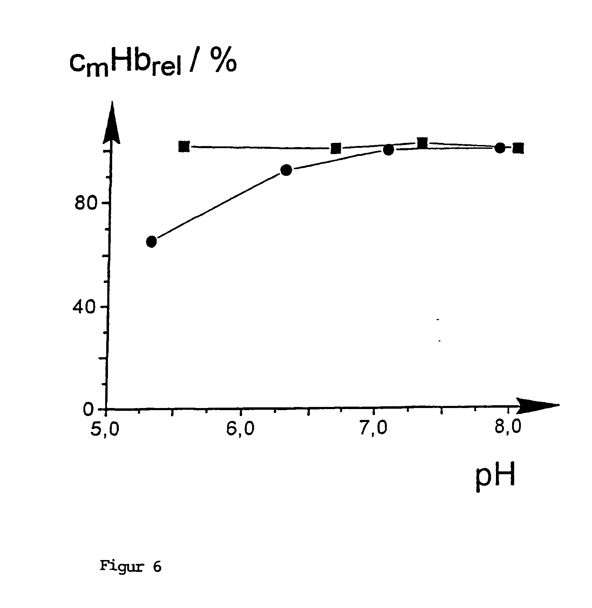

Die Herstellung der vernetzten Rinderhämoglobine erfolgte durch Vernetzen von hochreinem, konzentrierten Rinderhämoglobin mit einem 14-fachen molaren Überschuss Glutardialdehyd gemäß Beispiel 1, eine molekulare Fraktionierung der Syntheseprodukte, das Anbinden von mPEG-SPA-1000 und die präparative Vorbereitung zur *in* vitro-Fällungstitration gemäß Beispiel 2.
Eine Molekulargewichtsverteilung des nicht modifizierten Hämoglobin-Polymeren zeigt Abbildung 5, nämlich ein Eluogramm einer Volumenausschluss-Chromatographie (am Gel "Sephacryl S-400 HR", Pharmacia Biotech, Freiburg, D), der Modalwert der Molekulargewichtsverteilung beträgt hier 810 kg/mol.
Im *in vitro -* Fällungstest (die Ergebnisse zeigt Abbildung 6) wurden für das fraktionierte, nicht modifizierte Rinderhämoglobin-Polymer im pH-Intervall von 7,9 bis 5,3 Hämoglobinpolymer-Fällungen beobachtet. Die Probengefäße der mit PEG modifizierten fraktionierten Rinderhämoglobin-Polymeren mit pH-Werten zwischen 8,0 und 6,7 enthielten dagegen nach Zentrifugation keine Zentrifugate.
Durch das kovalente Anknüpfen von PEG-1000 wird die Plasmaverträglichkeit des vernetzten Rinderhämoglobins so erhöht, dass eine parenterale Verabreichung möglich ist, da mit Ausfällungen *in vivo* nicht gerechnet werden muss.

## Patentansprüche

1. Mit menschlichem und tierischem Plasma verträgliches Hämoglobin-Derivat, **dadurch gekennzeichnet, dass** das Hämoglobin mittels eines bifunktionellen Vernetzers für Proteine, ausgewählt aus Butandiepoxid, Divinylsulfon, einem Diisocyanat, einem Di-N-Hydroxysuccinimidylester, einem Diimidoester, oder einem Dialdehyd oder Glykolaldehyd, unter Einsatz eines bezogen auf monomeres Hämoglobin 3- bis 60-fachen molaren Überschusses kovalent vernetzt und anschließend an das Hämoglobin ein Polyalkylenoxid mit einem Molekulargewicht (molare Masse) zwischen 200 und 5.000 g/mol kovalent angeknüpft ist, welches ein Verknüpfungsprodukt des Polyalkylenoxids mit einem eine terminale Hydroxygruppe des Polyalkylenoxid maskierenden Molekül ist.

2. Hämoglobin- Derivat gemäß Anspruch 1, **dadurch gekennzeichnet, dass** das Diisocyanat ausgewählt ist aus Hexamethylendiisocyanat, Zyklohexyldiisocyanat, 2,5-Bisisocyanatobenzolsulfonsäure.

3. Hämoglobin- Derivat gemäß Anspruch 1, **dadurch gekennzeichnet, dass** der Dialdehyd ausgewählt ist aus Glyoxal oder Glutardialdehyd.

4. Mit Plasma verträgliches Hämoglobin-Derivat gemäß einem der Ansprüche 1 bis 3, **dadurch gekennzeichnet, dass** das Hämoglobin menschlichen Ursprungs, vom Rind oder vom Schwein ist

5. Mit Plasma verträgliches Hämoglobin-Derivat gemäß einem der Ansprüche 1 bis 4, **dadurch gekennzeichnet, dass** an das Hämoglobin ein Derivat eines Polyalkylenoxids, ausgewählt aus Polyethylenoxid, Polypropylenoxid, oder Kopolymere aus Ethylenoxid und Propylenoxid, geknüpft ist.

6. Hämoglobin-Derivat nach Anspruch 53, **dadurch gekennzeichnet, dass** die Anzahl der angeknüpften Polyalkylenoxide zwischen 1 und 40 Moleküle Polyalkylenoxid pro Molekül des Hämoglobinmonomeren beträgt.

7. Hämoglobin-Derivat nach Anspruch 64, **dadurch gekennzeichnet, dass** das Hämoglobin mittels Glutardialdehyd vernetzt ist.

8. Hämoglobin-Derivat gemäß einem der Ansprüche 1 bis 7, **dadurch gekennzeichnet, dass** das kovalent angeknüpfte Polyalkylenoxid eine molare Masse zwischen 500 und 2000 g /mol besitzt.

9. Verfahren zur Herstellung von Hämoglobin-Derivaten als künstliche Sauerstoffträger, die mit menschlichem/tierischem Blutplasma verträglich sind, **dadurch gekennzeichnet, dass** das Hämoglobin mit einem bifunktionellen Vernetzer für Proteine, ausgewählt aus Butandiepoxid, Divinylsulfon, einem Diisocyanat, einem Di-N-Hydroxysuccinimidylester, einem Diimidoester, oder einem Dialdehyd, oder Glykolaldehyd unter Einsatz eines bezogen auf monomeres Hämoglobin 3- bis 60-fachen molaren Überschusses kovalent vernetzt und an das vemetzte Hämoglobin ein Polyalkylenoxid mit einem Molekulargewicht (molare Masse) zwischen 200 und 5.000 g/mol, welches ein Verknüpfungsprodukt des Polyalkylenoxids mit einem eine terminale Hydroxygruppe des Polyalkylenoxid maskierenden Molekül ist, kovalent angeknüpft wird.

10. Verfahren nach einem der Ansprüche 9, **dadurch gekennzeichnet, dass** Hämoglobine vom Menschen, Rind oder Schwein eingesetzt werden.

11. Verfahren nach Anspruch 9 oder 10, **dadurch gekennzeichnet, dass** Hämoglobin in einer wässrigen Elektrolytlösung sowohl mit einem 3- bis 60-fachen molaren Überschuss, bezogen auf monomeres Hämoglobin, eines bifunktionellen Vernetzers für Proteine vernetzt, als auch mit einem 1- bis 40-fachen molaren Überschuss, bezogen auf monomers Hämoglobin, eines Polyalkylenoxids kovalent verknüpft wird und anschließend der Überschuss an Reaktanden entfernt und das Produkt gereinigt wird.

12. Verfahren nach einem der Ansprüche 9 bis 11, **dadurch gekennzeichnet, dass** das Diisocyanat, ausgewählt ist aus Hexamethylendiisocyanat, Zyklohexyldiisocyanat und 2,5-Bisisocyanatobenzolsulfonsäure.

13. Verfahren nach einem der Ansprüche 9-12, **dadurch gekennzeichnet, dass** der Dialdehyd ausgewählt ist aus Glyoxal, dem analog reagierenden Glykolaldehyd, oder Glutardialdehyd.

14. Verfahren nach Anspruch 13, **dadurch gekennzeichnet, dass** der bifunktionelle Vernetzer für Proteine Glutardialdehyd ist.

15. Verfahren nach einem der Ansprüche 9 bis 14 **dadurch gekennzeichnet, dass** als Polyalkylenoxid ein Derivat eines Polyethylenoxids, Polypropylenoxids, oder Kopolymeren aus Ethylenoxid und Propylenoxid eingesetzt wird.

16. Verwendung eines mit Plasma verträglichen vernetzten Hämoglobins gemäß einem der Ansprüche 1 bis 8 oder hergestellt gemäß einem der Ansprüche 9 bis 15 zur Herstellung eines Mittels zur intravasalen oder biomedizinischen Anwendung als künstlicher Sauerstoffträger.

17. Verwendung gemäß Anspruch 16, **dadurch gekennzeichnet, dass** das Mittel in Form einer pharmazeutischen Zubereitung als ein Ersatz des Blutes, oder als ein Zusatz zum Blut oder zu einer Nährlösung, im menschlichen und tierischen Organismus, in einzelnen Organen, oder in biotechnischen Anwendungen verwendet wird.

## Claims

1. A haemoglobin derivative compatible with human and animal plasma, **characterised in that** the haemoglobin is covalently crosslinked by means of a difunctional crosslinking agent for proteins selected from butane diepoxide, divinyl sulfone, a diisocyanate, a di-N-hydroxysuccinimidyl ester, a diimido ester, or a dialdehyde or glycol aldehyde, using a 3 to 60 times molar excess relative to monomeric haemoglobin and then a polyalkylene oxide with a molecular weight (a molar mass) of between 200 and 5,000 g/mol is covalently linked to the haemoglobin, which polyalkylene oxide is a linkage product of the polyalkylene oxide with a molecule which masks a terminal hydroxyl group of the polyalkylene oxide.

2. A haemoglobin derivative according to claim 1, **characterised in that** the diisocyanate is selected from hexamethylene diisocyanate, cyclohexyl diisocyanate, 2,5-bisisocyanatobenzenesulfonic acid.

3. A haemoglobin derivative according to claim 1, **characterised in that** the dialdehyde is selected from glyoxal or glutardialdehyde.

4. A plasma-compatible haemoglobin derivative with according to any one of claims 1 to 3, **characterised in that** the haemoglobin is of human, bovine or porcine origin.

5. A plasma-compatible haemoglobin derivative according to any one of claims 1 to 4, **characterised in that** a derivative of a polyalkylene oxide, selected from polyethylene oxide, polypropylene oxide, or copolymers of ethylene oxide and propylene oxide, is linked to the haemoglobin.

6. A haemoglobin derivative according to claim 5, **characterised in that** the number of linked polyalkylene oxides amounts to between 1 and 40 molecules of polyalkylene oxide per molecule of the haemoglobin monomer.

7. A haemoglobin derivative according to claim 6, **characterised in that** the haemoglobin is crosslinked by means of glutardialdehyde.

8. A haemoglobin derivative according to any one of claims 1 to 7, **characterised in that** the covalently linked polyalkylene oxide has a molar mass of between 500 and 2000 g/mol.

9. A method for producing haemoglobin derivatives as artificial oxygen carriers which are compatible with human/animal blood plasma, **characterised in that** the haemoglobin is covalently crosslinked with a difunctional crosslinking agent for proteins selected from butane diepoxide, divinyl sulfone, a diisocyanate, a di-N-hydroxysuccinimidyl ester, a diimido ester, or a dialdehyde, or glycol aldehyde using a 3 to 60 times molar excess relative to monomeric haemoglobin and a polyalkylene oxide with a molecular weight (a molar mass) of between 200 and 5,000 g/mol is covalently linked to the crosslinked haemoglobin, which polyalkylene oxide is a linkage product of the polyalkylene oxide with a molecule which masks a terminal hydroxyl group of the polyalkylene oxide.

10. A method according to claim 9, **characterised in that** human, bovine or porcine haemoglobin is used.

11. A method according to claim 9 or 10, **characterised in that** haemoglobin is both crosslinked in an aqueous electrolyte solution with a 3 to 60 times molar excess, relative to monomeric haemoglobin, of a difunctional crosslinking agent for proteins, and covalently linked with a 1 to 40 times molar excess, relative to monomeric haemoglobin, of a polyalkylene oxide and then the excess of reactants is removed and the product is purified.

12. A method according to any one of claims 9 to 11, **characterised in that** the diisocyanate is selected from hexamethylene diisocyanate, cyclohexyl diisocyanate and 2,5-bisisocyanatobenzenesulfonic acid.

13. A method according to any one of claims 9-12, **characterised in that** the dialdehyde is selected from glyoxal or glutardialdehyde.

14. A method according to claim 13, **characterised in that** the difunctional crosslinking agent for proteins is glutardialdehyde.

15. A method according to any one of claims 9 to 14, **characterised in that** a derivative of a polyethylene oxide, polypropylene oxide, or copolymer of ethylene oxide and propylene oxide is used as the polyalkylene oxide.

16. Use of a plasma-compatible crosslinked haemoglobin according to any one of claims 1 to 8 or produced according to any one of claims 9 to 15 for producing an agent for intravasal or biomedical use as an artificial oxygen carrier.

17. Use according to claim 16, **characterised in that** the agent is used in the form of a pharmaceutical preparation as a blood substitute, or as an additive to blood or to a nutrient solution, in the human and animal body, in individual organs, or in biotechnological applications.

## Revendications

1. Dérivé d'hémoglobine compatible avec du plasma humain et animal, **caractérisé en ce que** l'hémoglobine est réticulée par covalence au moyen d'un réticulant difonctionnel pour des protéines, choisi parmi le diépoxyde de butane, la divinylsulfone, un diisocyanate, un ester de di-N-hydroxysuccinimidyle, un diimidoester ou un dialdéhyde ou un glycolaldéhyde, avec utilisation d'un excès molaire d'un facteur 3 à 60 par rapport à l'hémoglobine monomère, puis un poly(oxyde d'alkylène) présentant un poids moléculaire (une masse moléculaire) entre 200 et 5000 g/mole, qui est un produit de liaison du poly(oxyde d'alkylène) avec une molécule masquant un groupe hydroxy terminal du poly(oxyde d'alkylène) est lié par covalence à l'hémoglobine.

2. Dérivé d'hémoglobine selon la revendication 1, **caractérisé en ce que** le diisocyanate, est choisi parmi l'hexaméthylènediisocyanate, le cyclohexyldiisocyanate, l'acide 2,5-bis-isocyanatobenzènesulfonique.

3. Dérivé d'hémoglobine selon la revendication 1, **caractérisé en ce que** le dialdéhyde est choisi parmi le glyoxal ou le glutardialdéhyde.

4. Dérivé d'hémoglobine compatible avec du plasma selon l'une quelconque des revendications 1 à 3, **caractérisé en ce que** l'hémoglobine est d'origine humaine, bovine ou porcine.

5. Dérivé d'hémoglobine compatible avec du plasma selon l'une quelconque des revendications 1 à 4, **caractérisé en ce qu'**un dérivé d'un poly(oxyde d'alkylène), choisi parmi le poly(oxyde d'éthylène), le poly(oxyde de propylène) ou les copolymères d'oxyde d'éthylène et d'oxyde de propylène est lié à hémoglobine.

6. Dérivé d'hémoglobine selon la revendication 5, **caractérisé en ce que** le nombre de poly(oxydes d'alkylène) liés est situé entre 1 et 40 molécules de poly(oxyde d'alkylène) par molécule de monomère d'hémoglobine.

7. Dérivé d'hémoglobine selon la revendication 6, **caractérisé en ce que** l'hémoglobine est réticulée au moyen de glutardialdéhyde.

8. Dérivé d'hémoglobine selon l'une quelconque des revendications 1 à 7, **caractérisé en ce que** le poly(oxyde d'alkylène) lié par covalence présente une masse molaire entre 500 et 2000 g/mole.

9. Procédé pour la préparation de dérivés d'hémoglobine comme transporteurs synthétiques d'oxygène, qui sont compatibles avec le plasma sanguin humain/animal, **caractérisé en ce que** l'hémoglobine est réticulée par covalence avec un réticulant difonctionnel pour des protéines, choisi parmi le diépoxyde de butane, la divinylsulfone, un diisocyanate, un ester de di-N-hydroxysuccinimidyle, un diimidoester ou un dialdéhyde ou un glycolaldéhyde, avec utilisation d'un excès molaire d'un facteur 3 à 60 par rapport à l'hémoglobine monomère et un poly(oxyde d'alkylène) présentant un poids moléculaire (une masse moléculaire) entre 200 et 5000 g/mole, qui est un produit de liaison du poly(oxyde d'alkylène) avec une molécule masquant un groupe hydroxy terminal du poly(oxyde d'alkylène) est lié par covalence à l'hémoglobine.

10. Procédé selon la revendication 9, **caractérisé en ce qu'**on utilise des hémoglobines d'hommes, bovine ou porcine.

11. Procédé selon la revendication 9 ou 10, **caractérisé en ce que** l'hémoglobine est réticulée dans une solution électrolytique aqueuse avec un excès molaire d'un facteur 3 à 60, par rapport à l'hémoglobine monomère, d'un réticulant difonctionnel pour des protéines ainsi que liée par covalence avec un excès molaire d'un facteur 1 à 40, par rapport à l'hémoglobine monomère, d'un poly(oxyde d'alkylène), puis l'excès de réactifs est éliminé et le produit est purifié.

12. Procédé selon l'une quelconque des revendications 9 à 11, **caractérisé en ce que** le diisocyanate est choisi parmi l'hexaméthylènediisocyanate, le cyclohoxyldiisocyanate et l'acide 2,5-bis-isocyanatobenzènesulfonique.

13. Procédé selon l'une quelconque des revendications 9 à 12, **caractérisé en ce que** le dialdéhyde est choisi parmi le glyoxal ou le glutardialdéhyde.

14. Procédé selon la revendication 13, **caractérisé en ce que** le réticulant difonctionnel pour les protéines est le glutardialdéhyde.

15. Procédé selon l'une quelconque des revendications 9 à 14, **caractérisé en ce qu'**on utilise comme poly(oxyde d'alkylène) un dérivé d'un poly(oxyde d'éthylène), d'un poly(oxyde de propylène) ou de copolymères d'oxyde d'éthylène et d'oxyde de propylène.

16. Utilisation d'une hémoglobine réticulée compatible avec du plasma selon l'une quelconque des revendications 1 à 8 ou préparée selon l'une quelconque des revendications 9 à 15 pour la préparation d'un agent destiné à une utilisation intravasculaire ou biomédicale comme transporteur synthétique d'oxygène.

17. Utilisation selon la revendication 16, **caractérisée en ce que** l'agent est utilisé sous forme d'une préparation pharmaceutique comme produit de remplacement du sang ou comme additif pour le sang ou une solution nutritive, dans l'organisme humain et animal, dans des organes particuliers ou dans des utilisations biotechniques.
